(19) Europäisches Patentamt
European Patent Office
Office européen des brevets

(11) EP 1 922 331 B1

(12) # FASCICULE DE BREVET EUROPEEN

(45) Date de publication et mention de la délivrance du brevet:
**06.07.2016 Bulletin 2016/27**

(21) Numéro de dépôt: **06794300.1**

(22) Date de dépôt: **07.08.2006**

(51) Int Cl.:
***C07K 14/415*** (2006.01) ***A61K 36/48*** (2006.01)
***A61K 8/44*** (2006.01) ***A61Q 5/00*** (2006.01)
***A61Q 5/02*** (2006.01) ***A61Q 5/06*** (2006.01)
***A61Q 5/12*** (2006.01) ***A61K 8/64*** (2006.01)
***A61K 8/73*** (2006.01) ***A61Q 19/00*** (2006.01)
***A61Q 19/10*** (2006.01)

(86) Numéro de dépôt international:
**PCT/FR2006/001914**

(87) Numéro de publication internationale:
**WO 2007/017591 (15.02.2007 Gazette 2007/07)**

(54) **COMPOSITION COMPRENANT UN EXTRAIT DE GUAR ET UTILISATION D'UN EXTRAIT DE GUAR COMME AGENT DE TRAITEMENT ET/OU DE MODIFICATION DE SURFACES**

GUAR EXTRACT ZUR BEHANDLUNG VON OBERFLÄCHEN

GUAR EXTRACT-CONTAINING COMPOSITION AND USE OF A GUAR EXTRACT IN THE FORM OF A SURFACE TREATING AND/OR MODIFYING AGENT

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **05.08.2005 FR 0508381**

(43) Date de publication de la demande:
**21.05.2008 Bulletin 2008/21**

(73) Titulaire: **RHODIA CHIMIE**
**93300 Aubervilliers (FR)**

(72) Inventeurs:
- **KARAGIANNI, Katerina**
  **F-75013 Paris (FR)**
- **MONIN, Vincent**
  **Plainsboro, NJ 08536 (US)**
- **SASSI, Jean-François**
  **22620 Ploubazlanec (FR)**

(74) Mandataire: **Cardon, Flavie et al**
**Rhodia Operations**
**Direction Propriété Industrielle**
**40, rue de la Haie-Coq**
**93306 Aubervilliers Cedex (FR)**

(56) Documents cités:
**WO-A-01/93810 FR-A- 2 437 829**

- **KHALIL M M: "BIOCHEMICAL AND TECHNOLOGICAL STUDIES ON THE PRODUCTION OF ISOLATED GUAR PROTEIN" DIE NAHRUNG, VCH VERLAGSGESELLSCHAFT, WEINHEIM, vol. 45, no. 1, 2001, pages 21-24, XP008021585 ISSN: 0027-769X cité dans la demande**
- **NATH J P ET AL: "Functional properties of guar proteins" JOURNAL OF FOOD SCIENCE, INSTITUTE OF FOOD TECHNOLOGISTS, CHICAGO, IL, US, vol. 46, no. 4, 1981, pages 1255-1259, XP002272557 ISSN: 0022-1147**
- **TRIMBLE R: "THE POSSIBLE UTILIZATION OF GUAR PROTEIN" PROCEEDINGS INTERNATIONAL CONGRESS FOOD SCIENCE AND TECHNOLOGY, vol. 1, 1983, pages 29-39, XP008028375**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 922 331 B1

## Description

La présente invention a pour objet une composition pour le traitement et/ou la modification de surfaces, par exemple une composition cosmétique ou pharmacologique ou phytosanitaire ou pour les soins ménagers comprenant un extrait de guar. Elle a également pour objet l'utilisation de l'extrait comme agent de traitement et/ou de modification de surfaces. La composition et l'utilisation présentent notamment un intérêt particulier dans le domaine de la cosmétique, notamment pour la réalisation de produits de coiffage, pour la mise en forme des cheveux, ou pour la réalisation de shampoings, après-shampoings ou gels douches, pour le conditionnement de la peau et/ou des cheveux. Elles présentent également un intérêt dans le domaine de la détergence, notamment pour les soins domestiques, et dans le domaine phytosanitaire.

L'utilisation d'extraits de guar est connue. Par exemple, on connaît l'utilisation de gomme de guar ou de dérivés de guar dans les domaines de la cosmétique et de l'alimentaire, notamment comme agent de modification de la rhéologie et/ou de la texture d'une composition ou d'un aliment, ou comme agent conditionneur sur la peau et/ou les cheveux.

Le document FR 2 437 829 décrit par exemple une composition cosmétique comprenant un extrait de guar.

Par ailleurs, les fractions de protéines contenues dans la gomme de guar ou dans les farines d'extraction de guar ont également été décrites (Anderson et al., Food Additives and Contaminants, 1985, vol. 2, N°4, 225-230 ; Nath et al., J. Agric. Food Chem., 1980, 28, 844-847). M. M. Khalil, (Production of Isolated Guar Protein, Food 45, 2001, N° 1, 21-24) s'est plus particulièrement intéressé aux qualités nutritionnelles des protéines isolées à partir de farines de graines de guar ("guar seed flour").

Il existe par ailleurs dans l'industrie un besoin constant pour de nouvelles compositions, par exemple comprenant de nouveaux produits, pouvant présenter de nouvelles propriétés ou améliorer des propriétés. En particulier, il existe un grand intérêt pour les produits d'origine végétale.

Il a maintenant été mis en évidence que l'extrait protéinique de guar et ses dérivés présentaient des propriétés bénéfiques sur la peau et les phanères, notamment les cheveux, qui les rendent particulièrement utiles en cosmétique de soin et en pharmacologie, en particulier dans les applications dermatologiques.

Ainsi, les compositions comprenant un extrait protéinique de guar peuvent présenter d'excellentes propriétés de conditionnement des cheveux ou de la peau, ainsi que des propriétés sensorielles ou cosmétiques intéressantes, qui peuvent être recherchées par les consommateurs. Ainsi, elles peuvent procurer un profil intéressant de douceur, de souplesse, de volume, de démêlage, d'aptitude au coiffage sur cheveux mouillé et/ou d'aptitude au coiffage sur cheveux sec, d'aptitude à réparer les cheveux. Ces effets peuvent rendre plus simples les formulations et/ou moins coûteuses. Ces compositions sont également particulièrement utiles pour réparer et/ou hydrater la peau. Par ailleurs, l'extrait présente une modularité de formulation (formulabilité) élevée. Cela peut rendre son utilisation simple et peu coûteuse.

Il a également été montré que ces composés peuvent être utilisés dans les compositions pour les soins ménagers (aussi bien pour les soins mis en oeuvre dans la sphère privée des consommateurs, que pour les soins mis en oeuvre dans une sphère publique comme le nettoyage industriel ou institutionnel des surfaces et textiles), en particulier des compositions détergentes, notamment pour le traitement, par exemple le nettoyage, des surfaces dures, dont la vaisselle, ou des surfaces textiles. De façon plus spécifique, ces compositions permettent d'adoucir et de faciliter le repassage des tissus. Elles permettent également de faciliter le nettoyage des surfaces dures.

De manière inattendue, il a en outre été mis en évidence qu'il était possible de diminuer le rebond des gouttes et d'accroître la rétention des formulations phytosanitaires et/ou d'éléments nutritionnels en introduisant des extraits protéiniques de guar ou des dérivés de ceux-ci dans lesdites formulations appliquées à des plantes. Les extraits protéiniques de guar et leurs dérivés possèdent un effet positif sur l'adhésion instantanée et, par conséquent, sur la rétention, dans des conditions de granulométrie élevée, des gouttes de pulvérisation. Avantageusement, ces extraits limitent significativement les phénomènes de rebond, usuellement observés lors de la pulvérisation sous forme de gouttes de granulométrie élevée et limitent par ailleurs le phénomène de ruissellement. Les extraits de protéines de guar ou leurs dérivés améliorent ainsi l'adhésion instantanée et, par conséquent, la rétention phytosanitaire et/ou d'éléments nutritionnels appliqués sous forme de gouttelettes sur les plantes à traiter.

Ainsi, selon un premier aspect, l'invention a pour objet une composition pour le traitement et/ou la modification de surfaces, comprenant un extrait protéinique de guar, le cas échéant sous forme d'un dérivé, ledit extrait protéinique comprenant au moins 65 % en poids de protéines.

La composition peut par exemple être:

- une composition cosmétique,
- une composition pharmacologique,
- une composition pour les soins ménagers, ou
- une composition phytosanitaire.

Selon un autre aspect, l'invention a pour objet l'utilisation de l'extrait protéinique de guar, le cas échéant sous forme d'un dérivé, en tant qu'agent de traitement et/ou de modification de surfaces.

Selon un autre aspect, l'invention a pour objet un procédé de traitement et/ou de modification de surfaces comprenant une étape d'application d'un composition comprenant l'extrait protéinique de guar, éventuellement sous forme d'un dérivé, sur une surface.

Définitions

Dans la présente demande, on désigne par guar la plante *Cyanopsis tetragonoloba.* Dans le présent exposé, les pourcentages en poids sont exprimés en poids sec, sauf mention contraire.

Dans la présente demande on désigne par "graines de guar" ("guar seeds" en anglais) les graines issues du guar. Les graines de guar comprennent l'écorce ("Hull" en anglais), plus ou moins fibreuse, le germe, et deux cupules de guar ("guar splits" on "endosperme halves" en anglais) qui constituent l'endosperme de guar. Les cupules (respectivement l'endosperme) sont (est) riche(s) en galactomannes. Les graines de guar sont constituées généralement de 35 à 40% en poids de l'endosperme, de 42 à 47% en poids du germe, et de 14 à 17 % en poids de l'écorce.

Dans la présente demande, on désigne par "farine de guar" ("guar flour" en anglais) ou par poudre de guar ("guar powder" en anglais), une poudre issue de l'endosperme de guar.

Dans la présente demande, on désigne par "guar natif" des chaînes macromoléculaires de type galactomannane issues de l'endosperme de guar, n'ayant pas subi de modification chimique par greffage de groupes chimiques. Le guar natif comprend des macromolécules comportant une chaîne principale d'unités D-mannopyranose liées en position bêta(1-4) substituée par des unités D-galactopyranose en position bêta(1-6). Le guar natif présente un rapport mannose/galactose d'environ 2. Le guar natif peut éventuellement avoir été partiellement dépolymérisé (abaissement de la masse moléculaire).

Dans la présente demande, on désigne par "gomme de guar" ("guar gum" en anglais) un produit essentiellement constitué de guar natif, sous forme de cupules guar ("guar splits") ou de farine ou de poudre de guar ("guar flour" ou "guar powder").

Le germe du guar comprend généralement de 35 à 45 % en poids de protéines, de 30 à 35% en poids de fibres, moins de 5% de galactommanes, environ 5% de sels, environ 5-10% d'eau, environ 6% de graisses, les pourcentages en poids étant exprimés par rapport au poids total de germe de guar. Le germe de guar ("churi" dans la langue utilisée dans les bassins de culture de l'Inde et du Pakistan notamment) est parfois désigné de manière impropre sous le terme de "protéine de guar". Dans la présente demande, le terme "protéine de guar" ne désigne pas le germe du guar.

Les cupules de guar comprennent environ 4 à 6% de matière protéinique.

L'écorce de guar ne comprend généralement pas de protéines (environ 0% en poids). L'écorce de guar est parfois désignée par "korma" dans la langue utilisée dans les bassins de culture de l'Inde et du Pakistan notamment.

La gomme de guar est obtenue par un procédé comprenant la séparation plus ou moins fine d'un produit comprenant les cupules de guar, d'une part, (avec éventuellement des impuretés) et d'un sous-produit comprenant l'écorce et le germe, d'autre part (avec éventuellement des impuretés). Le procédé est généralement essentiellement mécanique, mais des étapes de lavage et/ou d'extraction à l'aide d'eau ou de solvants ou d'agents gonflants, ainsi que des étapes de purification avec des agents acides ou basiques peuvent intervenir. Ces procédés, étapes, produits et sous-produits sont connus de l'homme du métier.

Dans la présente demande, on désigne par "farine d'extraction de guar" ("guar meal" en anglais), le sous-produit issu de la récupération des cupules, comprenant typiquement environ 70-80% en poids de germe de guar ("churi"), et environ 20-30% en poids d'écorce ("korma") et moins de 10% en poids d'endosperme.

Dans la présente demande, on désigne par "extrait protéinique de guar" ou "protéine de guar" un produit comprenant au moins 65% en poids de protéines, typiquement de 65 à 95% en poids, extraites du germe de guar, typiquement issu d'un procédé de concentration et/ou d'extraction et/ou d'isolement à partir de farine d'extraction de guar. Dans la présente demande, on pourra également se référer à un "isolat de protéine de guar" ("guar proteine isolate" en anglais) ou à un "concentré de protéine de guar" ("guar proteine concentrate" en anglais).

Dans la présente demande, sauf mention contraire, les quantités en poids de protéines sont déterminées à partir du taux d'azote mesuré selon la méthode Kjeldhal, connue. Cette méthode est par exemple décrite sur le lien suivant: http://www.rosesci.com/Products/Chemical%20Analysis/K eldahl%20 Chemistry%20-%20Overview.htm. Le taux d'azote est multiplié par 6,25 pour obtenir la quantité en poids de protéine.

La composition comprend un extrait protéinique de guar, éventuellement sous forme d'un dérivé, ledit extrait protéinique comprenant au moins 65%, de préférence de 65% à 95%, par exemple de 65% à 85%, en poids de protéines.

L'extrait protéinique de guar peut comprendre en outre des matières grasses, de l'eau, des sucres, et des sels minéraux.

La composition en acides aminés de la distribution de la masse moléculaire de l'extrait protéinique est susceptible de varier de manière plus ou moins importante en fonction de l'origine des graines de guar, de leur maturation,

des conditions d'extraction utilisées.

Parmi les acides aminés présents dans l'extrait protéinique de guar, on peut citer principalement l'acide glutamique (Glu), l'arginine (Arg), l'acide aspartique (Asp), la leucine (Leu), la glycine (Gly), la sérine (Ser) et la proline (Pro).

L'extrait protéinique de guar peut comprendre ainsi :

- de 10 % à 30 % d'acide glutamique, notamment de 15 % à 25 %;
- de 5% à 25 % d'arginine, notamment de 10 % à 20%, et plus particulièrement de 12 % à 16 % ;
- de 5 % à 20 % d'acide aspartique, notamment de 10 % à 15 %;
- de 1 % à 10 % de leucine, et notamment de 5 à 10 % ;
- de 1 % à 8% de glycine, et notamment de 4 % à 6 % ;

les pourcentages étant exprimés en masse par rapport à la masse totale d'acides aminés contenu dans l'extrait.

De préférence, l'extrait protéinique de guar comprend les compositions en acides aminés suivantes :

| Acides aminés | % |
|---|---|
| Cystéine | 1,38 |
| Méthionine | 1,19 |
| acide aspartique | 10,90 |
| Thréonine | 2,75 |
| Sérine | 4,83 |
| acide glutamique | 22,97 |
| Proline | 4,21 |
| Glycine | 5,19 |
| Alanine | 3,32 |
| Valine | 3,51 |
| Isoleucine | 3,18 |
| Leucine | 6,21 |
| Tyrosine | 3,70 |
| Phénylalanine | 4,14 |
| Lysine | 3,78 |
| Histidine | 2,89 |
| Arginine | 14,41 |
| Tryptophane | 1,44 |

les pourcentages étant exprimés en masse par rapport à la masse totale d'acides aminés de l'échantillon de la protéine isolée.

L'extrait protéinique de guar possède ainsi une quantité relativement importante d'arginine comparée aux protéines couramment utilisées dans le domaine cosmétique telles que les protéines de soja, les protéines de lait ou les protéines d'avoine. Or, l'arginine est un acide aminé particulièrement utile dans le domaine cosmétique car il possède par exemple une action hydratante sur la peau.

Dérivés

Les compositions selon l'invention peuvent comprendre un extrait protéinique de guar sous forme d'un dérivé.

L'extrait protéinique de guar sous forme d'un dérivé peut typiquement présenter la même répartition en acides aminés que l'extrait non dérivé, le cas échéant avec des masse molaires plus faibles.

Dans la présente demande, on désigne par "extrait protéinique de guar sous forme d'un dérivé" ou "extrait protéiniques de guar dérivé" ou "protéine de guar dérivée" un produit susceptible d'être obtenu par modification chimique des molécules de l'extrait protéinique de guar.

En d'autres termes, il s'agit

- d'un extrait protéinique comprenant des groupes, identiques ou différents, greffés de façon covalente sur des fonctions d'acides aminés compris dans l'extrait protéinique.

Sans vouloir se limiter à une quelconque théorie, les groupes chimiques sont susceptibles de se greffer notamment sur les fonctions -OH ou -$NH_2$ ou-COOH portées par les chaînes latérales des acides aminés et/ou les fonctions terminales des protéines.

Les groupes chimiques que l'on peut greffer sur les acides aminés compris dans l'extrait protéinique sont:

- les groupes cationiques ou cationisables. Par "groupements cationisables", on entend des groupements qui sont potentiellement cationiques, i.e. qui peuvent être rendus cationiques en fonction du pH du milieu.

Il est possible de combiner plusieurs modifications, par exemple une hydrolyse et un greffage. Il est possible de combiner des greffages de plusieurs groupes différents.

Dérivés cationiques ou potentiellement cationiques

Parmi les groupements cationiques ou cationisables, on peut citer les groupements comprenant des ammoniums quaternaires ou des amines tertiaires, des pyridiniums, des guanidiniums, des phosphoniums ou des sulfoniums.

Les produits cationiques selon l'invention peuvent être obtenus en faisant réagir de façon classique les protéines de l'extrait protéinique de guar telles quelles ou après qu'elles aient subi une hydrolyse enzymatique ou chimique de manière à cliver les liaisons peptidiques.

*Cationisation par substitution nucléophile*

L'introduction de groupements cationiques ou cationisables dans l'extrait protéinique de guar peut être réalisée par une réaction de substitution nucléophile.

Dans le cas où l'on souhaite introduire un groupement ammonium, le réactif adapté utilisé peut être :

- le chlorure de 3-chloro-2-hydroxypropyltriméthylammonium, vendu sous le nom de QUAB 188 par la société DEGUSSA ;
- un époxyde porteur d'un ammonium quaternaire tel que le chlorure de 2,3-époxypropyltriméthylammonium vendu sous le nom de QUAB 151 par la société DEGUSSA ou des composés analogues ;
- le chlorure de diéthylaminoéthyle ;

ou des accepteurs de Michaël comme, par exemple, des acrylates ou des méthacrylates porteurs d'ammonium quaternaires ou d'amines tertiaires.

*Cationisation par estérification*

L'introduction de groupements cationiques ou cationisables sur les acides aminés de l'extrait de protéines de guar peut être réalisée par une estérification avec des acides aminés tels que, par exemple, la glycine, la lysine, l'arginine, l'acide 6-aminocaproïque ou avec des dérivés d'acides aminés quaternisés tel que, par exemple, le chlorhydrate de bétaïne.

*Cationisation par polymérisation radicalaire*

L'introduction de groupements cationiques ou cationisables dans l'extrait protéinique de guar peut être réalisée par une polymérisation radicalaire comprenant le greffage de monomères comprenant au moins un groupement cationique ou cationisable sur les acides aminés de l'extrait protéique de guar.

L'amorçage radicalaire peut être effectué à l'aide de cérium comme cela est décrit dans la publication European Polymer Journal, vol. 12, p. 535-541, 1976. L'amorçage radicalaire peut également être effectué par un rayonnement ionisant et en particulier un bombardement sous faisceau d'électrons.

Les monomères comprenant au moins un groupement cationique ou cationisable mis en oeuvre pour réaliser cette polymérisation radicalaire peuvent être, par exemple, des monomères comprenant au moins une insaturation éthylénique et au moins un atome d'azote quaternaire ou quaternisable en ajustant le pH.

Parmi ces monomères comprenant au moins une insaturation éthylénique et au moins un atome d'azote quaternaire ou quaternisable en ajustant le pH, on peut citer les composés de formules (I), (II), (III), (IV) ou (V) suivants :

- le composé de formule générale (I)

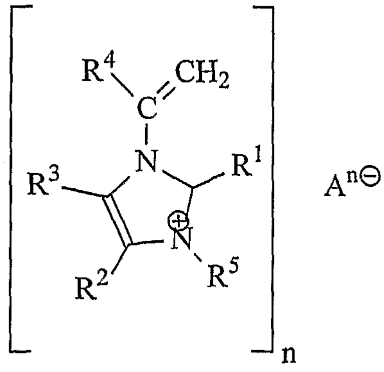

**(I)**

dans laquelle :

- $A^{n\ominus}$ représente un ion $Cl^{\ominus}$, $Br^{\ominus}$, $I^{\ominus}$, $SO_4^{2\ominus}$, $CO_3^{2\ominus}$, $CH_3\text{-}OSO_3^{\ominus}$, $OH^{\ominus}$ ou $CH_3\text{-}CH_2\text{-}OSO_3^{\ominus}$,
- $R^1$ à $R^5$ identiques ou différents représentent, indépendamment les uns des autres, un groupe alkyle ayant de 1 à 20 atomes de carbone, un radical benzyle ou un atome de H, et

n vaut 1 ou 2, ou

- le composé de formule générale (II)

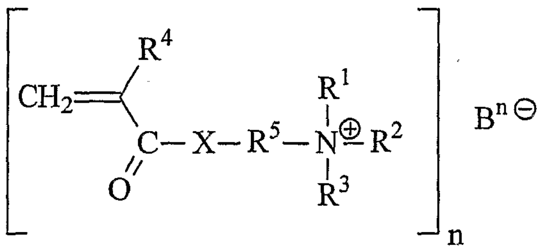

**(II)**

dans laquelle :

- X représente un groupe -NH ou un atome d'oxygène O,
- $R^4$ représente un atome d'hydrogène ou un groupe alkyle ayant de 1 à 20 atomes de carbone,
- $R^5$ représente un groupe alcène ayant de 1 à 20 atomes de carbone,
- $R^1$, $R^2$, $R^3$ identiques ou différents représentent, indépendamment les uns des autres, un groupe alkyle ayant de 1 à 20 atomes de carbone,
- $B^{n\ominus}$ représente un ion $Cl^{\ominus}$, $Br^{\ominus}$, $I^{\ominus}$, $SO_4^{2\ominus}$, $CO_3^{2\ominus}$, $CH_3\text{-}OSO_3^{\ominus}$, $OH^{\ominus}$ ou $CH_3\text{-}CH_2\text{-}OSO_3^{\ominus}$, et
- n vaut 1 ou 2, ou

- le composé de formule générale (III)

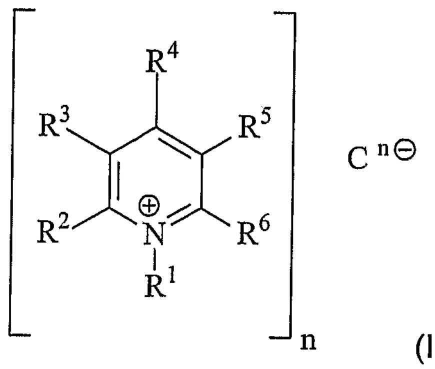

(III)

dans laquelle :

- $R^1$ à $R^6$ identiques ou différents représentent, indépendamment les uns des autres, un atome d'hydrogène ou un groupe alkyle ayant de 1 à 20 atomes de carbone, mais avec un des groupes $R^1$ à $R^6$ représentant un groupe $-CH = CH_2$,
- $C^{n\ominus}$ représente un ion $Cl^{\ominus}$, $Br^{\ominus}$, $I^{\ominus}$, $SO_4^{2\ominus}$, $CO_3^{2\ominus}$, $CH_3\text{-}OSO_3^{\ominus}$, $OH^{\ominus}$ ou $CH_3\text{-}CH_2\text{-}OSO_3^{\ominus}$, et
- n vaut 1 ou 2, ou

▪ le composé de formule générale (IV)

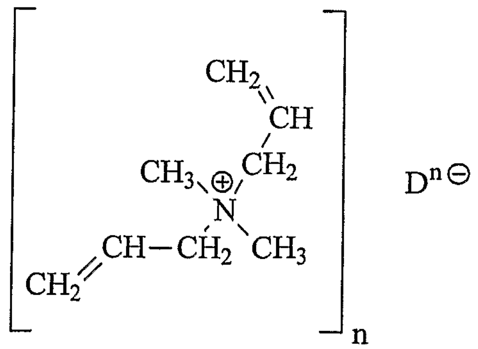

(IV)

dans laquelle :

- $D^{n\ominus}$ représente un ion $Cl^{\ominus}$, $Br^{\ominus}$, $I^{\ominus}$, $SO_4^{2\ominus}$, $CO_3^{2\ominus}$, $CH_3\text{-}OSO_3^{\ominus}$, $OH^{\ominus}$ ou $CH_3\text{-}CH_2\text{-}OSO_3^{\ominus}$, et

- n vaut 1 ou 2.

De préférence, les monomères comprenant au moins une insaturation éthylénique et au moins un atome d'azote quaternaire ou quaternisable sont choisis parmi :

- l'acrylate de 2-diméthylaminoéthyle (ADAM),
- l'acrylate de 2-diméthylaminoéthyle quaternisé (ADÀM-Quat),
- le méthacrylate de 2-diméthylaminoéthyle (MADAM),
- le méthacrylate de 2-diméthylaminoéthyle quaternisé (MADAM-Quat),
- le méthacrylate de 2-diéthylaminoéthyle quaternisé forme chlorure dénommé Pleximon 735 ou TMAE MC 80 par la société Röhm,
- le chlorure de diallyldiméthylammonium (DADMAC),
- le triméthyl ammonium propyl méthacrylamide forme chlorure dénommé MAPTAC, ou
- leurs mélanges.

L'extrait protéinique de guar dérivé cationique peut contenir des motifs cationiques ou cationisables issus d'une

transformation chimique après polymérisation de monomères précurseurs de fonctions cationiques ou cationisables. On peut citer à titre d'exemple du poly-p-chlorométhylstyrène qui, après réaction avec une aminé tertiaire telle qu'une triméthyl amine, forme du polyparatriméthylaminométhylstyrène quaternisé.

Les motifs cationiques ou cationisables sont associés avec des contre-ions chargés négativement. Ces contre-ions peuvent être choisis parmi les ions chlorures, bromures, iodures, fluorures, sulfates, méthylsulfates, phosphates, hydrogénophosphates, phosphonates, carbonates, hydrogénocarbonates, ou hydroxydes.

De préférence, on utilise des contre-ions choisis parmi les hydrogénophosphates, les méthylsulfates, les hydroxydes et les chlorures.

Le degré de substitution des extraits protéiniques de guar modifiés cationiques selon l'invention est d'au moins 0,01 et de préférence d'au moins 0.05.

Si le degré de substitution est inférieur à 0,01, l'efficacité de la fixation de l'extrait protéinique sur la surface à traiter peut être réduite.

Si le degré de substitution dépasse 0,05, l'efficacité en terme d'affinité pour la surface peut être nettement améliorée.

Le degré de substitution de l'extrait protéinique de guar modifié cationique correspond au nombre moyen de charges cationiques introduites par acide aminé. Ce degré de substitution peut être déterminé par analyse élémentaire, par exemple sur l'azote.

<u>Procédé d'obtention de l'extrait protéinique de guar</u>

L'extrait protéinique de guar peut être préparé à partir des graines de guar, ou de préférence à partir de farine d'extraction de guar (guar meal), selon les techniques usuelles d'extraction des protéines à partir de végétaux, notamment de protéines de soja. De telles techniques sont décrites dans l'encyclopédie Kirk Othmer, "Encyclopedia of Industrial Chemistry", vol. A22, pages 295 à 300 et pages 612 à 614.

L'extrait protéinique de guar peut notamment être isolé ou concentré à partir de la farine d'extraction de guar, laquelle est le sous-produit de la récupération des cupules des graines de guar. Cette farine d'extraction de guar est disponible dans le commerce. Elle est par exemple commercialisée par Rhodia sous le nom "Guar Meal" 100% ou 31%. Les farines d'extraction de guar peuvent être également préparées selon la méthode décrite par North J. P., Subramanian N., Narasinja Rao, M. S., J. Agric. Food Chem. 26 (5), 1243 (1978).

L'extrait protéinique de guar peut être préparé selon la méthode dite de « concentration ». Cette méthode comprend généralement:

a1) la mise en suspension de germes de guar, de préférence d'une farine d'extraction de guar, dans un liquide d'extraction ;
b1) la séparation de la phase solide S1 et la récupération de la phase liquide L1 ;
c1) l'ajustement du pH de la phase liquide L1 récupérée à un pH acide ;
d1) la récupération de l'extrait protéinique sous la forme d'un précipité.

Les germes de guar mis en oeuvre à l'étape a1) du procédé englobent les germes de guar qui peuvent être présents, par exemple dans les graines de guar éventuellement dépourvues de leur écorce et/ou broyées sous forme de poudre, dans les farines de guar ou encore dans les farines d'extraction de guar. De préférence, à l'étape a1), on procède à l'extraction de farines d'extraction guar.

Le liquide d'extraction à l'étape a1) peut être choisi parmi les solvants organiques, de l'eau ou un mélange de ceux-ci.

Parmi les solvants organiques utiles à titre de liquide d'extraction, on peut par exemple citer les alcools tels que l'éthanol, les solvants hydrocarbonés tels que le n-hexane, les éthers tels que le diéthyl éther.

Le liquide d'extraction peut être également de l'eau, de préférence déminéralisée, et plus préférentiellement une solution à pH basique, éventuellement en combinaison avec un co-solvant organique, tel qu'un alcool.

Les solutions à pH basique sont des solutions de pH > 7, notamment > 8, et plus particulièrement > 9. Il peut s'agir notamment de solutions d'hydroxyde de métal alcalin telles que des solutions d'hydroxyde de sodium ou de potassium.

Le milieu d'extraction peut, en outre, contenir des sels minéraux tels que le chlorure de sodium ou de potassium.

La concentration des sels minéraux dans le milieu d'extraction peut varier dans une large mesure et est généralement comprise entre 0,5 M et 1,5 M.

L'extraction peut avoir lieu dans une vaste gamme de températures, notamment entre 20°C et 80°C, de préférence entre 40°C et 60°C, et plus préférentiellement à 55°C environ.

L'extraction peut être réalisée avec une proportion de farine d'extraction de guar:liquide d'extraction comprise entre 1:100 à 50:100 exprimée en poids, de préférence avec une proportion comprise entre 2:100 et 25:100 exprimée

en poids.

Le temps requis pour l'extraction peut également varier considérablement selon de nombreux facteurs, notamment la température de l'extraction et le liquide d'extraction. Une durée généralement comprise entre 10 mn et 3 heures s'avère généralement suffisante.

L'extrait brut obtenu à l'étape a1) est ensuite séparé par exemple par filtration ou centrifugation. La phase solide S1, moins riche en protéines et qui peut par exemple contenir de l'endosperme et/ou l'écorce, est éliminée, et la phase liquide L1, correspondant à l'extrait, riche en protéines, est récupérée.

Selon une variante, préalablement à l'étape c1), le solide S1 est récupéré à l'issue de l'étape b1) et extrait à son tour avec un liquide d'extraction qui peut être identique ou différent du liquide d'extraction mis en oeuvre avec les germes de guar à l'étape a1). L'extrait brut obtenu est ensuite séparé, et la phase liquide L2 est récupérée.

Les/la phase(s) liquide(s) extraite(s) L1 ou (L1 + L2) est/sont ensuite acidifiée(s) par addition d'une solution concentrée d'un acide minéral tel que l'acide chlorhydrique. Une quantité suffisante de cet acide est additionnée de façon à ce que le pH du liquide L1 ou L1+L2 soit ajusté à une valeur $\leq 7$, en particulier $\leq 5$, et plus particulièrement $\leq 4$.

Le précipité qui se forme à la suite de cette opération est récupéré, par exemple par centrifugation ou filtration.

Il peut être ensuite séché, par exemple par concentration sous vide, atomisation ou lyophilisation. Il est à noter qu'une fraction particulière du précipité obtenu peut être purifiée ou concentrée par extraction liquide/liquide au moyen de solvants organiques ou par chromatographie préparative.

Selon une variante intéressante, l'extrait protéinique de guar est préparé à partir des farines d'extractions de guar ou de farines de guar selon la technique dite « d'isolation ». Cette méthode comprend les étapes mises en oeuvres dans l'étape de concentration à la différence que les germes de guar mis en oeuvre à l'étape a1) sont sous la forme de farines de guar ou d'extraction de guar préalablement enrichis en protéines selon les étapes de :

a2) tamisage des farines de guar ou d'extraction de guar et récupération des particules de diamètre inférieur à 1500 $\mu$m, notamment à 1400 $\mu$m ;
b2) séparation et récupération des particules les plus lourdes contenues dans la farine de guar ou d'extraction de guar tamisée.

Au cours de l'étape a2), une partie importante de l'endosperme de guar, pauvre en protéine, est éliminée.

L'étape b2) peut être réalisée selon des techniques conventionnelles, par exemple au moyen d'un sécheur à lit fluidisé, équipé d'un dispositif pour collecter les particules les plus légères entraînées par un courant d'air. Cette étape permet ainsi l'élimination des particules légères, riches en fibres, et la récupération des particules les plus denses qui sont généralement plus riches en protéines.

L'extrait protéinique obtenu peut être utilisé tel que résultant du procédé d'extraction, lequel peut conduire à une dépolymérisation, i.e. une hydrolyse partielle des protéines.

En variante, de façon subséquente à l'étape d1), le procédé peut comprendre en outre une réaction d'hydrolyse chimique ou enzymatique des liaisons peptidiques. Selon une réalisation préférée, les protéines ont des masses moléculaires inférieures à 30 000 Da, notamment de 100 à 30 000, de préférence entre 500 et 20 000, et de façon plus préférentielle de l'ordre de 750 à 15 000 Da, lesquelles sont particulièrement préférées dans le domaine des produits cosmétiques. Les protéines hydrolysées sont en effet généralement plus substantives et pénètrent plus facilement dans le cortex du cheveu ou dans l'épiderme.

Parmi les enzymes utiles pour cette étape de dépolymérisation des protéines, on peut citer, par exemple, les protéases d'origine animale, végétales, microbiennes ou fongique.

Comme exemples de réactifs chimiques utiles pour cette étape de dépolymérisation, on peut citer les bases minérales telles que les hydroxydes de métal alcalin ou alcalino-terreux, les acides minéraux tels que l'acide chlorhydrique.

L'extrait protéinique de guar dérivé peut être préparé selon le procédé comprenant les étapes suivantes :

a) préparation d'un extrait protéinique de guar ;
b) réaction de greffons sur des fonctions d'acides aminés compris dans l'extrait protéinique ; et éventuellement
c) récupération du produit obtenu.

L'extrait protéinique de guar, mis en oeuvre à l'étape a), peut être préparé selon l'une des méthodes décrites ci-dessus.

Des techniques pour l'étape b) ont été décrites plus haut.

Les réactions des greffons sur les fonctions portées par les acides aminés contenus dans l'extrait protéinique de guar peuvent être réalisées par l'application ou l'adaptation de procédés de substitution nucléophile, d'estérification ou de polymérisations radicalaires utilisés jusque là ou décrits dans la littérature, par exemple ceux décrits dans R.C. Larock, Comprehensive Organic Transformations, VCH Publishers, 1989.

Dans le cas où le dérivé est un hydrolysat, l'étape b) n'est pas mise en oeuvre. On peut mettre en oeuvre les hydrolyses telles que décrites ci-dessus.

Compositions - Utilisations

La composition pour la modification et/ou le traitement de surfaces comprend l'extrait protéinique de guar, éventuellement sous forme d'un dérivé, et généralement d'autres ingrédients (ou "composants"). En particulier elle comprend généralement un vecteur, le plus souvent liquide, appelé vecteur d'application topique pour les compositions cosmétiques ou pharmacologiques.

Ainsi des compositions utiles peuvent être des compositions pour le traitement et/ou la modification de surfaces comprenant:

- un vecteur liquide, par exemple un vecteur aqueux, alcoolique ou hydroxyalcoolique, et
- l'extrait protéinique de guar, éventuellement sous forme d'un dérivé,
- éventuellement au moins un tensioactif, par exemple un tensioactif anionique, non ionique, amphotère, ou un mélange,
- éventuellement d'autres ingrédients.

La composition peut être utilisée dans un procédé de traitement ou de modification d'une surface, comprenant les étapes suivantes:

- appliquer sur la surface la composition, et
- éventuellement, éliminer le vecteur ou diluer la composition ou modifier le pH.

Les surfaces visées peuvent notamment être:

- la peau et/ou les cheveux pour les compositions cosmétiques,
- les dents pour des compositions pour soins buccaux dentaires,
- les surfaces dures, dont la vaisselle, pour des compositions pour les soins ménagers, par exemple
    - les surfaces (métal, céramique, plastic, verre...) de la vaisselle, des formulations destinées au nettoyage de la vaisselle à la main ou en machine,
    - les sols (bois, céramique, plastic, béton...) pour des compositions de nettoyage multi-usage ou de nettoyage des sols,
    - les surfaces présentes dans les cuisines pour des compositions de nettoyage multi-usage ou de nettoyage des cuisines,
    - les surfaces présentes dans les salles de bain pour des compositions de nettoyage multi-usage ou de nettoyage des salles de bain,
    - les vitres ou pare-brise, pour des compositions de nettoyage des vitre ou des pare-brise
- la peau pour des compositions pharmacologiques,
- les feuilles des plantes pour des compositions phytosanitaires.
- les surfaces textiles pour les compositions de nettoyage et/ou de rinçage (par exemple des assouplissants) et/ou de repassage du linge.

L'application d'une composition cosmétique selon l'invention est de préférence effectuée par voie topique.

La composition est destinée plus particulièrement au traitement de la peau ou du cheveu et peut se présenter sous forme d'onguent, de crème, d'huile, de lait, de pommade, de poudre, de tampon imbibé, de solution, de fluide, de gel, de spray, de lotion, de suspension, d'un produit moulé (un savon par exemple), de mousse. Les compositions cosmétiques selon l'invention peuvent ainsi se présenter sous la forme d'émulsion simple huile-dans-eau ou eau-dans-huile, d'émulsion multiple, de microémulsion, de gel aqueux ou hydroalcoolique.

Il peut notamment s'agir d'un shampooing, d'un après shampooing (rinçé ou non rinçé), d'un produit de coiffage (pour la mise en forme des cheveux par exemple) ou d'un gel douche.

Les compositions cosmétiques peuvent être des compositions pour le soin et l'hygiène de la peau et/ou du cheveu.

Des exemples de compositions cosmétiques pour cheveu, sont notamment des compositions de shampooing, d'après-shampooing, de produit de coiffage, de produits protecteurs, réparateurs, adoucissants ou encore de produits pour permanente et coloration.

Sans vouloir se limiter à une quelconque théorie, il a été observé que les extraits protéiniques de guar ou leurs dérivés présentent une très bonne affinité pour le cheveu, ce qui pourrait expliquer leurs propriétés de fixation importantes, et notamment leur résistance à l'humidité sur cheveux secs.

Des exemples de compositions cosmétiques pour la peau sont notamment des produits pour le visage et le corps, des produits de jour ou de nuit, des produits solaires, des produits d'hygiène anti-âge ou anti-rides, des produits anti-pollution, des gels douches, des crèmes pour les mains.

Les compositions cosmétiques selon l'invention peuvent comprendre de 0,0001 à 4% dudit extrait par rapport au poids de la composition, de préférence de 0,01 à 1%.

Les protéines de guar contenues dans les compositions cosmétiques selon l'invention peuvent ainsi représenter de 0,00003% à 4 % en poids, notamment de 0,001% à 1 % en poids de la composition cosmétique.

Des exemples de compositions détergentes sont notamment des compositions à usage domestique et/ou de soins ménagers comme les produits pour le traitement des textiles tels que les lessives, les assouplissants, des produits d'entretien du linge, ou encore des produits de traitement des surfaces dures tels que les produits de nettoyage ou d'entretien des sols.

Les extraits protéiniques de guar ou leurs dérivés selon l'invention sont particulièrement utiles pour le traitement des phanères, tels que le cheveu ou la peau ou encore des textiles ou des surfaces ménagères, ou bien encore des plantes, en particulier de la surface foliaire des plantes.

Les extraits protéiniques de guar ou leurs dérivés peuvent être utilisés en association avec un véhicule cosmétiquement acceptable dans des compositions destinées à soigner et/ou réparer et/ou protéger la peau, les cheveux ou le cuir chevelu.

Les extraits protéiniques de guar ou leurs dérivés peuvent ainsi être utilisés en association avec un véhicule cosmétiquement acceptable, pour augmenter ou améliorer l'hydratation, l'élasticité, l'aspect satiné de la peau, ou encore pour raffermir la peau.

Ils peuvent également être utilisés en association avec un véhicule cosmétiquement acceptable, dans des compositions antipelliculaires, repousse du cheveu, antichute du cheveu, des compositions de soin du cheveu hydratantes, nourrissantes, revitalisantes. Ils peuvent être utilisés dans des compositions destinées à la protection du cheveu contre les agressions dues au froid, au soleil ou à la pollution (compositions dites « winter care », « summer care », anti-pollution). Enfin, ils peuvent être utilisés dans des compositions destinées à apporter du volume, de la brillance, de l'éclat à la couleur naturelle ou à la coloration, un toucher agréable, du ressort aux boucles, un effet lissant.

Par "véhicule cosmétiquement acceptable", on entend un véhicule adapté pour une utilisation en contact avec des cellules humaines et animales, en particulier des cellules de l'épiderme, sans toxicité, irritation, réponse allergique indue et similaire, et proportionné à un rapport avantage / risque raisonnable.

Les extraits protéiniques de guar ou leurs dérivés peuvent être utilisés dans les lessives, les assouplissants, les produits d'entretien pour la maison, par exemple les produits de nettoyage pour les sols ou les surfaces domestiques, et les produits anti-poussière.

De façon avantageuse, les produits selon l'invention présentent un effet adoucissant et anti-froissage s'agissant du traitement des textiles, ou encore un effet anti-traces, anti-salissures s'agissant des surfaces ménagères.

Sans vouloir se limiter à une quelconque théorie, l'application des produits selon l'invention conduirait à hydrophiliser les surfaces textiles ou ménagères, ce qui permettrait d'empêcher la formation de traces au séchage et de faciliter le nettoyage suivant.

Les extraits protéiniques de guar ou leurs dérivés peuvent être utilisés dans une composition phytosanitaire et/ou d'éléments nutritionnels destinée à être pulvérisée sur la surface foliaire des plantes, en tant qu'agent anti-rebond. Cet agent anti-rebond permet avantageusement d'améliorer l'adhésion instantanée et, par conséquent, la rétention et donc l'efficacité de la composition pulvérisée.

Des exemples de compositions phytosanitaires sont des formulations contenant une matière active telle qu'un herbicide, défanant, débroussaillant, bactéricide, fongicide, insecticide, acaricide, régulateur de croissance.

Ainsi, les extraits protéiniques de guar ou leurs dérivés permettent de limiter la perte au sol des compositions pulvérisées, laquelle est susceptible de générer une pollution des sols et des nappes phréatiques souterraines.

Dans les compositions, l'extrait protéinique de guar, le cas échéant sous forme d'un dérivé, peut avoir un effet de stabilisant de mousse, notamment dans des compositions cosmétiques moussantes, ou dans des compositions de nettoyage de la vaisselle ou du linge à la main.

D'autres détails ou avantages pourront apparaître au vu des exemples qui suivent.

<u>Exemple 1: Préparation d'un extrait protéiniques de guar "protéine de guar"</u>

<u>1. Enrichissement en protéines par traitement mécanique</u>

On utilise comme matière première du guar meal (farine d'extraction de guar) «31% mode » fourni par l'usine

Rhodia de Vernon, USA. Ce produit contient 34% en poids de protéines (= % Azote mesuré selon la méthode Kjeldhal × 6,25).

On tamise manuellement 1 kg de guar meal « 31% mode » pour retirer les particules supérieures à 1400 μm. Cette étape permet d'éliminer 116 g d'endosperme de guar qui sont pauvres en protéines.

Les 884 g de produit restant sont ensuite placés dans un sécheur à lit fluidisé de laboratoire (Retsch TG1) équipé d'un dispositif pour collecter les particules les plus légères entraînées par le courant d'air. Il est généralement admis que les parties de la graine riches en protéines (par exemple les morceaux de germes) sont plus denses. Ce dispositif permet donc d'isoler les particules les plus riches en protéines en éliminant les particules riches en fibre plus légères.

Après séparation à l'aide du sécheur, on récupère 625 g de particules lourdes (A) et 259 g de poussière. Les particules lourdes contiennent 39,2 % de protéines (= % Azote mesuré selon la méthode Kjeldhal × 6,25). Des essais de séparation précédents ont permis d'obtenir un produit contenant jusqu'à 42% de protéines.

2. Extraction des protéines

Dans un bécher de 5 L contenant 2280 g d'eau déminéralisée chauffée à 55°C, on ajoute 120 g de guar meal (A) enrichi en protéines selon le procédé détaillé ci-dessus. Le pH est ajusté à 10 à l'aide de soude 30 %. Cette suspension est agitée pendant 1 heure, en maintenant la température à 55°C.

La suspension est ensuite centrifugée 5 minutes à 3000 g. On récupère ainsi 2491 g de liquide (1) et 391,4 g de solide (2).

Le solide (2) est à nouveau suspendu dans de l'eau à 55°C pour préparer 3 kg de suspension. Le pH est aux alentours de 7. On ajoute de la soude 30 % pour relever le pH à 10, et on maintient 1 heure sous agitation à 55°C.

Cette suspension est également centrifugée 5 minutes à 3000 g. On récupère ainsi 2504,3 g de liquide (3) et 386 g de solide (4).

Les 2 liquides surnageants (1) et (3) obtenus précédemment sont mélangés dans un bécher de 5 L. Le pH est abaissé à 4,3 à l'aide d'acide chlorhydrique 35%, ce qui entraîne la précipitation des protéines. Cette suspension est centrifugée 10 minutes à 4200 g. On récupère 4772 g de liquide (5) et 135,5 g de solide (6).

Le solide (6) est séché en étuve sous vide (40°C, ~30 mm Hg) pendant 24 heures.

Le produit obtenu contient 7,4 % d'eau et 73,8 % de protéines (= % Azote mesuré selon la méthode Kjeldhal × 6,25).

Exemple 2: Préparation d'un extrait protéiniques de guar "protéine de guar"

On prépare une suspension contenant 10 % de guar meal en dispersant 45,3 kg de guar meal (Rhodia, usine de Vernon) dans 454 L d'eau.préchauffée à 55°C. Le pH initial de la suspension est de 4,85. On ajoute 1980 mL de soude 30 % pour relever le pH à 9,53. La suspension est maintenue 45 minutes à 55°C sous agitation.

La suspension est centrifugée, et on récupère 394,4 kg de liquide (1) et 107,7 kg de solide (2). Le solide (2) n'est pas réutilisé.

Le pH du liquide (1) est de 8,62. On ajoute 3920 mL d'acide chlorhydrique 30% pour abaisser le pH à 4,54, ce qui entraîne la précipitation des protéines. Cette suspension est maintenue 30 minutes à 45°C sous agitation.

La suspension est centrifugée. Le solide (3) est récupéré et on élimine 310,5 kg de liquide (4).

Le solide (3) est à nouveau suspendu dans de l'eau pour être lavé. On ajoute une quantité d'eau environ identique à la masse de solide (3). Le pH de cette suspension est de 4,75.

Cette suspension est centrifugée. On récupère 46,3 kg de solide (4) et 110,7 kg de liquide (5). Le liquide (5) n'est pas réutilisé.

On ajoute 510 mL de soude 30% au solide humide (4) pour amener le pH à 6,94.

Ce solide est ensuite pasteurisé par traitement thermique à 90°C pendant 20 secondes, puis atomisé. On obtient ainsi 6,8 kg de protéines de guar isolées.

L'échantillon de la protéine isolée contient :

71-72% protéines
5,6% cendres (calcination)
6,6% matières grasses (hydrolyse / extraction)
8,8% teneur en eau (Karl Fischer)]

Sucres (HPLC/Réfractométrie)

Fructose <0,1%
Glucose <0,1%
Saccharose 0,3%
Maltose <0,5%
Lactose <0,5%

Aminogramme

Acide aminé % en masse sur le contenu total en acides aminés de l'échantillon de la protéine isolée.

| *Acides aminés* | % |
|---|---|
| cystéine | 1,38 |
| méthionine | 1,19 |
| acide aspartique | 10,90 |
| thréonine | 2,75 |
| serine | 4,83 |
| acide glutamique | 22,97 |
| proline | 4,21 |
| glycine | 5,19 |
| alanine | 3,32 |
| valine | 3,51 |
| isoleucine | 3,18 |
| leucine | 6,21 |
| tyrosine | 3,70 |
| phénylalanine | 4,14 |
| lysine | 3,78 |
| histidine | 2,89 |
| arginine | 14,41 |
| tryptophan | 1,44 |

Acide aminé % en masse sur l'échantillon entier de protéine isolée.

| | |
|---|---|
| cystéine | 0,98 |
| méthionine | 0,84 |
| acide aspartique | 7,72 |
| thréonine | 1,95 |
| serine | 3,42 |
| acide glutamique | 16,27 |
| proline | 2,98 |
| glycine | 3,68 |
| alanine | 2,35 |
| valine | 2,49 |
| isoleucine | 2,25 |
| leucine | 4,4 |
| tyrosine | 2,62 |
| phénylalanine | 2,93 |
| lysine | 2,68 |
| histidine | 2,05 |
| arginine | 10,21 |
| tryptophane | 1,02 |

Masse moléculaire de la protéine : 13133 Da (Analyse MALDI-TOF-MS).
Métaux lourds : As+Cd+Cr+ Ni+Hg+Pb+Se+Sn <15 ppm.

Exemple 3 : Préparation d'un extrait protéinique de guar dérivé, cationisé

Modification d'un extrait protéinique de guar pour introduire des groupements cationiques de type triméthylammonium. On utilise comme composé de départ l'extrait protéinique de guar de l'exemple 2.

Dans un réacteur en verre double-enveloppe de 1 litre équipé d'une agitation mécanique et d'un réfrigérant ascendant, on introduit 160 ml d'eau déminéralisée, puis 0,75 g d'hydroxyde de sodium en pastilles. L'agitation est mise en marche à 50 tours par minutes pour dissoudre la soude solide. Une fois l'hydroxyde de sodium dissout, on ajoute 30 g de poudre d'extrait protéinique de guar présentant un taux d'humidité de 7,3% en masse.

Le réacteur est porté à 60°C masse au moyen d'une circulation de fluide caloporteur chaud dans la double enveloppe. Après 1 heure à 60°C sous agitation, on ajoute en goutte à goutte sur 20 minutes un volume de 66 ml de Quab® 151 (solution de chlorure de 2,3-époxypropyltriméthylammonium à 70% en masse dans l'eau, commercialisée par la société Degussa). Après addition, le mélange réactionnel est maintenu sous agitation à 60°C pendant 5 heures.

Après refroidissement et retour à température ambiante, on ajoute au milieu réactionnel de l'acide acétique glacial jusqu'à atteindre un pH égal à 7.

Le contenu du réacteur est transvasé dans une ampoule à décanter et additionné en goutte à goutte à 2 litres d'éthanol absolu agricole placés sous agitation. Un précipité apparaît. Ce solide est lavé par une succession de 3 séquences d'opérations de décantation, élimination du surnageant, remise en suspension dans 1,5 litre d'éthanol frais. En final, le solide est essoré sur entonnoir filtrant en verre fritté de porosité 2. Il est séché pendant 16 heures à 45°C sous vide 200 mbar compensé à 180 mbar avec de l'azote. On obtient en final 21,6 g de solide en poudre.

Les exemples 4 à 6 ont été réalisés avec les extraits protéiniques obtenus à l'exemple 2 (ci-après désignés « protéine de guar naturel ») et à l'exemple 3 (ci-après désignés « protéine de guar cationisée »).

Exemple 4 : Propriétés de l'extrait protéinique de guar et de l'extrait protéinique de guar cationisé

a) Point isoélectrique de l'extrait protéinique de guar naturel et cationisé (absence de sel)

Le point isoélectrique de l'extrait protéinique obtenu à l'exemple 2 a été déterminé en mesurant la transmittance au moyen d'un spectrophotomètre UV-Vis à 600 nm, en fonction du pH.

On prépare une solution à 1% d'extrait protéinique dans l'eau distillée. Le pH de la solution est de 7,3. Dès qu'on abaisse le pH, la solution devient laiteuse. A pH=4,9 on observe une précipitation : on a alors atteint le point isoélectrique de la protéine, c'est-à-dire le pH pour lequel la charge globale de la protéine est nulle. Il faut abaisser le pH jusqu'à 2,8 pour commencer à redissoudre la protéine, due à la charge globale cationique de la protéine. Pour des pH basiques, la turbidité diminue, du fait de l'augmentation de la charge globale négative de la protéine.

Le point isoélectrique de l'extrait protéinique cationisé de l'exemple 3 a été déterminé en mesurant la turbidité de la solution au moyen d'un spectrophotomètre UV-Vis à 600 nm, en fonction du pH mesuré par un pHmètre.

On réalise un diagramme qui montre l'influence du pH sur la turbidité et donc la solubilité d'une solution à 0,5% en protéine de guar cationisée dans l'eau déminéralisée.

Aucune précipitation n'est observée mais à un pH de 11,4 la solution devient très turbide et la transmittance est alors nulle. Puis, en augmentant encore le pH, la solution devient plus claire. Le point isoélectrique de la protéine cationisée se trouve déplacé aux pH plus élevés (à pH =11,4 environ) du fait de la cationisation.

Le point isoélectrique de l'extrait protéinique cationisé se situe donc aux environs de pH = 11,4.

b) Tension superficielle

L'évolution dans le temps de la tension superficielle d'une solution à 0,5% d'extrait protéinique de guar naturel ou cationisé dans l'eau distillée a été mesurée avec un tensiomètre à goutte pendante, pendant 300s. L'abaissement de la tension superficielle est rapide. Les valeurs à l'équilibre se situent vers 45mN/m (en iso-concentration, on a environ la même valeur d'équilibre pour des protéines animales comme la gélatine ou la bêta-lactoglobuline).

c) Emulsification

L'émulsification d'une huile d'orange a été réalisée : à pH neutre, 2,5% de protéines de guar naturel ou cationisé permettent d'émulsifier 10% d'huile avec l'Ultra Turrax et conduisent à une taille d'émulsion de 4 μm environ.

Exemple 5 : Shampooings et formulabilité

La formulation classique utilisée comprend les composants suivants :

- 0,3% d'extrait protéinique de guar naturel ou cationisé ;
- 2% de tensioactif amphotère ;
- 14% de tensioactif anionique ;
- 1-2% de sel NaCl ;
- de l'eau jusqu'à atteindre 100% de formulation.

Les tensioactifs utilisés :

CAPB : cocamidopropyl bétaine (tensioactif amphotère) ;
SLES : lauryléthersulfate de sodium (tensioactif anionique).

Mode opératoire

Le mode opératoire pour obtenir une formulation de shampooing appropriée est le suivant :

- mélanger l'extrait protéinique de guar naturel de l'exemple 2 ou modifié de l'exemple 3 dans l'eau dans un bécher, agiter jusqu'à dissolution (durée très variable suivant le polymère, peut nécessiter une modification du pH) ;
- ajouter le sel, agiter jusqu'à dissolution ;
- pendant ce temps, mélanger les deux tensioactifs dans un autre bécher pendant 30 minutes ;
- verser l'eau qui contient le sel et le polymère dans le bécher contenant les tensioactifs. Agiter 2 h ;
- ajuster le pH entre 5,5 et 6,5 avec de la soude ou de l'acide citrique.

Mesure de la viscosité

La viscosité des shampooings a été mesurée à l'aide d'un viscosimètre de type Brookfield. Un mobile (mobile 4) est plongé dans le shampooing et mis en rotation à la vitesse de 12 tours par minute à 25°C.

Stabilité dans le temps

Un échantillon dans un flacon en verre hermétique est mis dans une étuve à 45°C pendant 3 mois pour accélérer son vieillissement.

Transmittance

La transmittance a été mesurée dans une cellule de 1 cm avec un spectrophotomètre W-Vis à une longueur d'onde de 600 nm.
Les résultats sont les suivants :

| Polymère | % NaCl | Viscosité (cp) | Couleur | Transmittance (%) | Floculation | Stabilité |
|---|---|---|---|---|---|---|
| Protéine de guar | 2 | 4800 | Marron-jaune | 51,0 | non | 3 mois ok |
| Protéine de guar cationisée | 2 | 7940 | Marron-jaune | 60,3 | non | 3 mois ok |

Les protéines de guar "naturelles" ou cationisées permettent d'obtenir des formulations présentant une viscosité appropriée pour un shampooing. La viscosité d'un shampooing doit se situer généralement entre 2000 et 15000 cp.
Bien que les shampooings obtenus soient transparents, la transmittance en % n'est pas très élevée du fait que les protéines de guar naturelles ou cationisées, légèrement colorées, absorbent la lumière à 600 nm.
Les compositions de shampooing préparées ici peuvent être utiles, par exemple, pour apporter de la brillance, pour réparer et/ou protéger le cheveu, pour protéger et/ou fixer la couleur.

*Tableau 1 : Transmittance T(%) de la formulation en fonction du facteur de dilution*

| *Facteur de dilution* | *T% (5min) Protéine de guar* | *T% (5min) Protéine de guar cationisée* |
|---|---|---|
| 2,1 | 73,2 | 77,3 |
| 3,9 | 83,7 | 86,6 |
| 6,1 | 89,1 | 93,1 |
| 8,0 | 96 | 100 |

(suite)

| *Facteur de dilution* | *T% (5min) Protéine de guar* | *T% (5min) Protéine de guar cationisée* |
|---|---|---|
| 9,8 | 98,9 | 100 |
| 15,0 | 100 | 100 |

Exemple 6 : Produits de coiffage

a) Réparation d'un produit de coiffage contenant l'extrait protéinique de guar

Produits utilisés :

- Polymères épaississants (selon la formulation : gel, mousse ou spray) : Carbomer C (Carbomer C® (Rhodia))
- Produit fixant : l'extrait protéinique de guar, naturel ou cationisé
- Autres composants :

  AMP (Aminométhylpropanol) 90%, neutralise les charges ;
  Kathon® CG, conservateur.

Mode opératoire :

Une solution mère de polymère épaississant contenant également l'AMP d'une part, et une solution mère du produit fixant dans l'eau, d'autre part, sont préparées.

Dans un bécher de 100 mL muni d'une pale cadre, sont versés l'eau, la solution de polymère épaississant et AMP, la solution de produit fixant, dans les proportions définies au tableau ci-dessous. Deux gouttes de conservateur sont ensuite ajoutées, puis le pH est ajusté entre 5,5 et 7,5.

| Nature ou Fonction | Composant | % MA |
|---|---|---|
| Solvant | Eau | 99,54 |
| Polymère épaississant | Carbomère | 0,2 |
| Ajusteur de pH, neutralise les charges (-) | Aminométhylpropanol | 0,16 |
| Produit fixant | Extrait protéinique de guar naturel ou cationisé | 0,1 |
| Conservateur | Kathon CG | 2 gouttes |
| %MA représente le pourcentage en masse de matière active. | | |

La stabilité des formulations dans le temps a été évaluée en étudiant le vieillissement accéléré à l'étuve à 45°C dans un flacon en verre muni d'un bouchon bien étanche. Durée : 3 mois.

Le pH, ajusté entre 5,5 et 7,5, a été mesuré au pHmètre.

La viscosité : les formulations réalisées pour les différents polymères viscosants déterminent la viscosité et ainsi le conditionnement du gel :

Conditionnement en pot ($\eta$>30000ep).
Formulation en pompe (5000<$\eta$<30000cp).
Formulation en spray ($\eta$<5000cp).

La transparence de la solution a été mesurée avec un turbidimètre (662 photometer, Metrohm) à 600 nm. Les gels obtenus étant colorés, on a donc une mesure de la couleur en même temps que la transparence à 600 nm.

| Polymère épaississant | Produit fixant | Conditionnement | Stabilité |
|---|---|---|---|
| Carbomère | Extrait protéinique de guar | Spray | Stable |

(suite)

| Polymère épaississant | Produit fixant | Conditionnement | Stabilité |
|---|---|---|---|
| Carbomère | Protéine guar cationisée | Spray | Stable |

b) Test de résistance sous humidité contrôlée

La résistance sous humidité contrôlée des formulations à 0,2% de carbomère et 0,1% d'extrait de protéine de guar naturel ou cationisé a été testée dans les conditions définies ci-dessous.

Le test consiste à appliquer sur des mèches de cheveux naturelles calibrées une quantité contrôlée de gel ; on utilise trois mèches de cheveux pour l'étude de la reproductibilité.

Une fois les mèches préparées, elles sont placées dans une pièce à température ambiante et humidité ambiante pour être séchées pendant environ 2 h, elles sont positionnées verticalement afin qu'il n'y pas de déformation.

Une fois les mèches sèches, elles sont placées horizontalement dans une étuve à 21 °C et 90% d'humidité, puis on mesure l'évolution de l'angle d'inclinaison (par rapport à l'horizontal) à t = 5 min, 15 min, 30 min, 1h, 2h, 3 h et 4h.

Le pourcentage de tenue au cours du temps de la mèche a été mesuré selon l'équation :

$$((90° - \alpha) / (90° - \alpha_0)) * 100$$

$\alpha$ = angle d'inclinaison à t
$\alpha_0$ = angle d'inclinaison à $t_0$ (= 0 pour toutes les formulations)
t = temps où la mesure de l'angle est réalisée

Cette résistance à l'humidité a été comparée avec différentes formulations comparables à celles que l'on trouve dans le commerce, comprenant :

- 0,4% de polymère épaississant, et
- 0,3 % de produit fixant : PQ4 (Celquat® H100 de National Starch), PQ11 (Gafquat® d'ISP), PVP (PolyVinylPyrrolidone, ISP), PQ46 (Luviquat® Hold de BASF), PVP/VA (ISP).

*Tableau 2 : Résistance de la fixation par les produits de coiffage sous humidité contrôlée (21°C, 90% d'humidité)*

| | % moyen de tenue de la mèche | | | | | | |
|---|---|---|---|---|---|---|---|
| Temps (mn) | 0,1% de protéine de guar cationisée + 0,2% de polymère épaissisant | 0,1% d'extrait de protéine de guar naturel + 0,2% de polymère épaissisant | 0,3% de Gafquat® (PQ11 ISP) + 0,4% de polymère épaissisant | 0,3% Celquat® H100 + 0,4% de polymère épaissisant | 0,3% PVP + 0,4% de polymère épaissisant | 0,3% Luviquat® Hold + 0,4% de polymère épaissisant | 0,3% de PVP/VA +0,4% de polymère épaissisant |
| 0 | 100,00 | 100,00 | 100,00 | 100,00 | 100,00 | 100,00 | 100,00 |
| 5 | 98,89 | 94,81 | 96,30 | 93,06 | 93,33 | 97,04 | 94,07 |
| 15 | 86,85 | 86,30 | 88,33 | 86,94 | 76,67 | 85,93 | 76,85 |
| 30 | 73,52 | 77,78 | 67,78 | 60,28 | 57,78 | 67,41 | 57,04 |
| 60 | 66,85 | 72,41 | 57,22 | 51,94 | 52,04 | 57,22 | 52,04 |
| 120 | 64,81 | 70,19 | 47,41 | 46,11 | 46,30 | 46,67 | 46,67 |
| 180 | 64,81 | 70,00 | 43,70 | 46,11 | 43,70 | 42,04 | 44,44 |
| 240 | 64,81 | 69,26 | 42,04 | 45,00 | 42,22 | 39,81 | 42,59 |

Les résultats obtenus rapportés dans le tableau 2 montrent que, malgré la faible concentration de produit fixant (0,1 % de protéine de guar naturel ou cationisé), une très bonne résistance des formulations à l'humidité, supérieure à 60%, même après 4 heures, est obtenue.

En revanche, le pourcentage de tenue de la mèche obtenu avec les polymères fixants PQ4, PQ11, PQ46, PVP et PVP/VA est inférieur à 60 % après 60 mn bien que les proportions de carbomère et de polymère fixant utilisées soient plus importantes.

En utilisant 0,2% de polymère épaississant et 0,1 % de produit fixant, les pourcentages de tenue de la mèche obtenus avec les produits fixants PQ4, PQ11, PQ46, PVP et PVP/VA sont encore moindres.

## Revendications

1. Utilisation non-thérapeutique d'une composition comprenant un extrait protéinique de guar ou un extrait protéinique de guar comprenant des groupes greffés sur les fonctions d'acides aminés compris dans ledit extrait, ledit extrait comprenant au moins 65 % en poids de protéines, pour le traitement et/ou la modification de surfaces, ladite composition étant une composition cosmétique, une composition pour les soins ménagers ou une composition phytosanitaire, lesdites surfaces étant les cheveux et/ou la peau, les surfaces dures, la surface foliaire des plantes ou les surfaces textiles,lesdits groupes greffés, identiques ou différents, comprenant des groupes cationiques ou cationisables.

2. Utilisation selon la revendication 1, dans laquelle l'extrait protéinique comprend de 65 à 95 % en poids de protéines.

3. Utilisation selon la revendication 2, dans laquelle l'extrait protéinique comprend de 65 à 85 % en poids de protéines.

4. Utilisation selon l'une quelconque des revendications 1 à 3, dans laquelle l'extrait protéinique comprend :

   - de 10 % à 30 % d'acide glutamique ;
   - de 5 % à 25 % d'arginine,
   - de 5 % à 20 % d'acide aspartique ;
   - de 1 % à 10 % de leucine ;
   - de 1 % à 8% de glycine ;

   les pourcentages étant exprimés en masse par rapport à la masse totale d'acides aminés contenus dans l'extrait.

5. Utilisation selon l'une quelconque des revendications 1 à 4, dans laquelle les protéines ont des masses moléculaires inférieures à 30 000 Da.

6. Utilisation selon l'une quelconque des revendications 1 à 5, dans laquelle l'extrait est obtenu par extraction et/ou concentration et/ou isolement à partir de farines d'extraction de guar.

7. Utilisation selon l'une des revendications 1 à 6, dans laquelle les groupes cationiques ou cationisables sont choisis parmi des groupements comprenant des ammoniums quaternaires ou des amines tertiaires, des pyridiniums, des guanidiniums, des phosphoniums ou des sulfoniums.

8. Utilisation selon la revendication 7, dans laquelle les groupes cationiques ou cationisables sont choisis parmi des groupements comprenant des ammoniums quaternaires.

9. Utilisation selon les revendications 7 ou 8, dans laquelle les groupes cationiques ou cationisables sont associés avec des contre-ions chargés négativement choisis parmi les ions chlorures, bromures, iodures, fluorures, sulfates, méthylsulfates, phosphates, hydrogénophosphates, phosphonates, carbonates, hydrogénocarbonates, ou hydroxydes.

10. Utilisation selon l'une des revendications 1 à 9, ladite composition se présentant sous la forme d'un onguent, d'une crème, d'une huile, d'un lait, d'une pommade, d'une poudre, d'un tampon imbibé, d'une solution, d'un fluide, d'un gel, d'un spray, d'une lotion, d'une suspension, d'un produit moulé, ou d'une mousse.

11. Utilisation selon l'une des revendications 1 à 9, ladite composition étant une composition cosmétique sous la forme d'un shampoing, d'un gel douche, d'un après-shampoing, ou d'un produit de coiffage.

12. Utilisation selon l'une des revendications 1 à 9, ladite composition étant une composition pour les soins ménagers sous la forme d'une lessive, d'un assouplissant, d'un produit de nettoyage des surfaces dures, dont la vaisselle.

13. Utilisation d'un extrait protéinique de guar ou d'un extrait protéinique de guar comprenant des groupes greffés sur des fonctions d'acides aminés compris dans ledit extrait, comme agent cosmétique, ledit extrait comprenant au moins 65 % en poids de protéines, lesdits groupes greffés, identiques ou différents, comprenant des groupes cationiques ou cationisables.

14. Utilisation selon la revendication 13, comme agent cosmétique pour soigner et/ou réparer et/ou protéger les cheveux et/ou la peau.

15. Utilisation d'un extrait protéinique de guar ou d'un extrait protéinique de guar comprenant des groupes greffés sur des fonctions d'acides aminés compris dans ledit extrait pour la préparation d'une composition pharmaceutique destinée à soigner et/ou réparer et/ou protéger les cheveux et/ou la peau, ledit extrait comprenant au moins 65 % en poids de protéines, lesdits groupes greffés, identiques ou différents, comprenant des groupes cationiques ou cationisables.

16. Utilisation selon la revendication 13, comme agent fixant dans une composition de coiffage.

17. Composition pharmaceutique comprenant un extrait protéinique de guar ou un extrait protéinique de guar comprenant des groupes greffés sur des fonctions d'acides aminés compris dans ledit extrait, ledit extrait comprenant au moins 65 % en poids de protéines, lesdits groupes greffés, identiques ou différents, comprenant des groupes cationiques ou cationisables.

## Patentansprüche

1. Nichttherapeutische Verwendung einer Zusammensetzung, umfassend einen Guar-Proteinextrakt oder einen Guar-Proteinextrakt, der auf die Funktionen von in dem Extrakt vorhandenen Aminosäuren gepfropfte Gruppen umfasst, wobei der Extrakt mindestens 65 Gew.-% Proteine umfasst, zur Behandlung und/oder Modifikation von Oberflächen, wobei die Zusammensetzung eine Kosmetikzusammensetzung, eine Zusammensetzung für den Haushalt oder eine Pflanzenschutzzusammensetzung ist,
wobei es sich bei den Oberflächen um die Haare und/oder die Haut, harte Oberflächen, die Blattoberfläche von Pflanzen oder Textiloberflächen handelt, wobei die gepfropften Gruppen, die gleich oder verschieden sind, kationische oder kationisierbare Gruppen umfassen.

2. Verwendung nach Anspruch 1, wobei der Proteinextrakt 65 bis 95 Gew.-% Proteine umfasst.

3. Verwendung nach Anspruch 2, wobei der Proteinextrakt 65 bis 85 Gew.-% Proteine umfasst.

4. Verwendung nach einem der Ansprüche 1 bis 3, wobei der Proteinextrakt Folgendes umfasst:

- 10% bis 30% Glutaminsäure,
- 5% bis 25% Arginin,
- 5% bis 20% der Asparaginsäure,
- 1% bis 10% Leucin,
- 1% bis 8% Glycin,

wobei die Prozentsätze als Massenprozent, bezogen auf die Gesamtmasse an in dem Extrakt enthaltenen Aminosäuren, ausgedrückt sind.

5. Verwendung nach einem der Ansprüche 1 bis 4, wobei die Proteine Molekülmassen von weniger als 30 000 Da haben.

6. Verwendung nach einem der Ansprüche 1 bis 5, wobei der Extrakt durch Extraktion und/oder Aufkonzentration und/oder Isolation aus Guar-Extraktionsmehlen erhalten wird.

7. Verwendung nach einem der Ansprüche 1 bis 6, wobei die kationischen oder kationisierbaren Gruppen aus Gruppen ausgewählt sind, die quaternäre Ammoniumgruppen oder tertiäre Amine, Pyridiniumgruppen, Guanidiniumgruppen,

Phosphoniumgruppen oder Sulfoniumgruppen umfassen.

8. Verwendung nach Anspruch 7, wobei die kationischen oder kationisierbaren Gruppen aus Gruppen ausgewählt sind, die quaternäre Ammoniumgruppen umfassen.

9. Verwendung nach den Ansprüchen 7 oder 8, wobei die kationischen oder kationisierbaren Gruppen mit negativ geladenen Gegenionen assoziiert sind, die aus Chlorid-, Bromid-, Iodid-, Fluorid-, Sulfat-, Methylsulfat-, Phosphat-, Hydrogenphosphat-, Phosphonat-, Carbonat-, Hydrogencarbonat- oder Hydroxidionen ausgewählt sind.

10. Verwendung nach einem der Ansprüche 1 bis 9, wobei die Zusammensetzung in Form einer Salbe, einer Creme, eines Öls, einer Milch, einer Wundsalbe, eines Pulvers, eines getränkten Bausches, einer Lösung, eines Fluids, eines Gels, eines Sprays, einer Lotion, einer Suspension, eines gegossenen Produkts oder eines Schaums vorliegt.

11. Verwendung nach einem der Ansprüche 1 bis 9, wobei die Zusammensetzung eine Kosmetikzusammensetzung in Form eines Shampoos, eines Duschgels, einer Haarspülung oder eines Haarstylingprodukts ist.

12. Verwendung nach einem der Ansprüche 1 bis 9, wobei die Zusammensetzung eine Haushaltszusammensetzung in Form eines Waschmittels, eines Weichspülers, eines Reinigungsprodukts für harte Oberflächen, wie Geschirr, ist.

13. Verwendung eines Guar-Proteinextrakts oder eines Guar-Proteinextrakts, der auf die Funktionen von in dem Extrakt vorhandenen Aminosäuren gepfropfte Gruppen umfasst, als kosmetisches Mittel, wobei der Extrakt mindestens 65 Gew.-% Proteine umfasst, wobei die gepfropften Gruppen, die gleich oder verschieden sind, kationische oder kationisierbare Gruppen umfassen.

14. Verwendung nach Anspruch 13 als kosmetisches Mittel zur Pflege und/oder zur Reparatur und/oder zum Schutz der Haare und/oder der Haut.

15. Verwendung eines Guar-Proteinextrakts oder eines Guar-Proteinextrakts, der auf die Funktionen von in dem Extrakt vorhandenen Aminosäuren gepfropfte Gruppen umfasst, zur Herstellung einer pharmazeutischen Zusammensetzung zur Pflege und/oder zur Reparatur und/oder zum Schutz der Haare und/oder der Haut, wobei der Extrakt mindestens 65 Gew.-% Proteine umfasst, wobei die gepfropften Gruppen, die gleich oder verschieden sind, kationische oder kationisierbare Gruppen umfassen.

16. Verwendung nach Anspruch 13 als Festiger in einer Haarstylingzusammensetzung.

17. Pharmazeutische Zusammensetzung, umfassend einen Guar-Proteinextrakt oder einen Guar-Proteinextrakt, der auf die Funktionen von in dem Extrakt vorhandenen Aminosäuren gepfropfte Gruppen umfasst, wobei der Extrakt mindestens 65 Gew.-% Proteine umfasst, wobei die gepfropften Gruppen, die gleich oder verschieden sind, kationische oder kationisierbare Gruppen umfassen.

## Claims

1. Non-therapeutic use of a composition comprising a guar protein extract or a guar protein extract comprising groups grafted onto the functions of amino acids contained in said protein extract, said extract comprising at least 65% by weight of proteins, for treating and/or modifying surfaces,
said composition being a cosmetic composition, a composition for household care, or a phytosanitary composition, said surfaces being the hair and/or the skin, hard surfaces, leafy surfaces of plants or textile surfaces, said grafted groups, which are identical or different, comprising cationic or cationizable groups.

2. Use according to Claim 1, wherein the protein extract comprises from 65 to 95% by weight of proteins.

3. Use according to Claim 2, wherein the protein extract comprises from 65 to 85% by weight of proteins.

4. Use according to any one of Claims 1 to 3, wherein the protein extract comprises:

- from 10% to 30% glutamic acid;
- from 5% to 25% arginine;

- from 5% to 20% aspartic acid;
- from 1% to 10% leucine;
- from 1% to 8% glycine;

the percentages being expressed by weight relative to the total weight of amino acids contained in the extract.

5. Use according to any one of Claims 1 to 4, wherein the proteins have molecular weights lower than 30 000 Da.

6. Use according to any one of Claims 1 to 5, wherein the extract is obtained by extraction and/or concentration and/or isolation from guar extraction flours.

7. Use according to one of Claims 1 to 6, wherein the cationic or cationizable groups are selected from groups comprising quaternary ammoniums or tertiary amines, pyridiniums, guanidiniums, phosphoniums or sulphoniums.

8. Use according to Claim 7, wherein the cationic or cationizable groups are selected from groups comprising quaternary ammoniums.

9. Use according to Claim 7 or 8, wherein the cationic or cationizable groups are associated with negatively charged counterions selected from chloride, bromide, iodide, fluoride, sulphate, methylsulphate, phosphate, hydrogen phosphate, phosphonate, carbonate, hydrogen carbonate or hydroxide ions.

10. Use according to one of Claims 1 to 9, said composition being in the form of a salve, a cream, an oil, a milk, an ointment, a powder, an impregnated pad, a solution, a fluid, a gel, a spray, a lotion, a suspension, a moulded product or a foam.

11. Use according to one of Claims 1 to 9, said composition being a cosmetic composition in the form of a shampoo, a shower gel, a conditioner or a hairstyling product.

12. Use according to one of Claims 1 to 9, said composition being a composition for household care, in the form of a detergent, a fabric softener, or a product for cleaning hard surfaces, including crockery.

13. Use, as a cosmetic agent, of a guar protein extract or of a guar protein extract comprising groups grafted onto functions of amino acids contained in said extract, said extract comprising at least 65% by weight of proteins, said grafted groups, which are identical or different, comprising cationic or cationizable groups.

14. Use according to Claim 13, as a cosmetic agent, for caring for and/or repairing and/or protecting the hair and/or the skin.

15. Use of a guar protein extract or of a guar protein extract comprising groups grafted onto functions of amino acids contained in said extract, for the preparation of a pharmaceutical composition intended for caring for and/or repairing and/or protecting the hair and/or the skin, said extract comprising at least 65% by weight of proteins, said grafted groups, which are identical or different, comprising cationic or cationizable groups.

16. Use according to Claim 13, as fixing agent in a hairstyling composition.

17. Pharmaceutical composition comprising a guar protein extract or a guar protein extract comprising groups grafted onto functions of amino acids contained in said extract,
said extract comprising at least 65% by weight of proteins,
said grafted groups, which are identical or different, comprising cationic or cationizable groups.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2437829


### Littérature non-brevet citée dans la description


- **ANDERSON et al.** *Food Additives and Contaminants,* 1985, vol. 2 (4), 225-230
- **NATH et al.** *J. Agric. Food Chem.,* 1980, vol. 28, 844-847
- **M. M. KHALIL.** Production of Isolated Guar Protein. *Food,* 2001, vol. 45 (1), 21-24
- *European Polymer Journal,* 1976, vol. 12, 535-541
- **KIRK OTHMER.** Encyclopedia of Industrial Chemistry. vol. A22, 295-300, 612-614
- **NORTH J. P. ; SUBRAMANIAN N. ; NARASINJA RAO, M. S.** *J. Agric. Food Chem.,* 1978, vol. 26 (5), 1243
- **R.C. LAROCK.** Comprehensive Organic Transformations. VCH Publishers, 1989